# EUROPEAN PATENT SPECIFICATION

(11) **EP 1 404 357 B1**
(45) Date of publication and mention of the grant of the patent: **23.12.2009**
(21) Application number: 02749000.2
(22) Date of filing: 08.07.2002
(51) Int. Cl.: A61K 38/08, A61P 5/04

(54) **GONADOTROPIN RELEASING HORMONE ANTAGONIST IN GEL-FORMING CONCENTRATIONS**
GONADOTROPIN RELEASING HORMONE ANTAGONIST IN GEL-FORMENDEN KONZENTRATIONEN
ANTAGONISTE DE L'HORMONE DE LIBERATION DES GONADOTROPHINES DANS DES CONCENTRATIONS PERMETTANT LA GELIFICATION

(30) Priority: 12.07.2001 GB 0117057
(43) Date of publication of application: 07.04.2004
(73) Proprietor: Ferring BV, 2132 JX Hoofddorp (NL)
(72) Inventor: LÜCK, Martin, 2900 Hellerup (DK); BROQUA, Pierre, 92160 Antony (FR)
(74) Representative: HOFFMANN EITLE
(86) International application number: PCT/GB2002/003116
(87) International publication number: WO 2003/006049

(56) References cited:
- FR-A- 2 776 520
- US-A- 5 595 760
- US-A- 5 925 730
- JIANG G ET AL: "BETIDAMINO ACID-SCAN OF THE GNRH ANTAGONIST ACYLINE" JOURNAL OF MEDICINAL CHEMISTRY, AMERICAN CHEMICAL SOCIETY. WASHINGTON, US, vol. 40, 1997, pages 3739-3748, XP002071836 ISSN: 0022-2623

### SEQUENCE LISTING

<110> Ferring BV
<120> Pharmaceutical composition
<130> 43315wo01
<140>
   <141>
<150> GB 0117057.0 <151> 2001-07-12
<160> 1
<170> PatentIn Ver. 2.1
<210> 1
   <211> 10
   <212> PRT
   <213> Artificial Sequence
<220>
   <221> SITE
   <222> (1)
   <223> Xaa in 1 position is acetyl DNal
<220>
   <221> SITE
   <222> (2)
   <223> Xaa in 2 position is DCpa
<220>
   <221> SITE
   <222> (3)
   <223> Xaa in 3 position is DPal
<220>
   <221> SITE
   <222> (5)
   <223> Xaa in 5 position is 4-aminophenylanaline with omega amino group substituent X1, wher X1 is D- or L- Hor
<220>
   <221> SITE
   <222> (6)
<223> Xaa in 6 position is D-4-aminophenylalanine wherein the omega amino group has substitiuent X2, wherein x2 is a carbamoyl group
<220>
   <221> SITE
   <222> (8)
   <223> Lys in 8 position is isopropyllysine
<220>
   <223> Description of Artificial Sequence: Synthetic peptide
<400> 1
   Xaa Xaa Xaa Ser Xaa Xaa Leu Lys Pro Ala
     1               5                  10

## Description

The present invention relates to a pharmaceutical composition for the administration of a GnRH antagonist peptide useful in the treatment of sex hormone-dependent diseases.

### BACKGROUND

The discovery and characterization of GnRH (gonadotropin releasing hormone, previously Iuteinizing hormone releasing hormone, LHRH) as the first mediator in the hypothalamic-pituitary-gonadal axis has opened up new possibilities for the treatment of sex hormone-dependent conditions such as prostate cancer and precocious puberty. A first generation of therapeutic agents were the GnRH superagonists. These acted by continuously stimulating the GnRH receptor, leading to desensitization of the pathway. However, these agents tend to provoke a "flare" reaction and so are being displaced by a second generation, the GnRH antagonists.

A problem arises due to the need for chronic administration of the therapeutic agents. Like the superagonists before them, the current generation of GnRH antagonists are peptides that are unsuited for oral administration. Subcutaneous or intramuscular injection works well with the compounds, but daily injections would not be acceptable to the patient population and so current research is aimed at developing depot formulations of the antagonists. For the superagonists such depot technology is well established. The peptide is released from a biodegradable polymer matrix over a period of (typically) one to three months. The transfer of this technology to the antagonists is complicated by the need to administer larger quantities of drug substance. As a result, there has been a significant effort made to develop antagonists that are more potent (so requiring less drug substance to be included in the depot) or that have physicochemical properties compatible with higher drug/polymer ratios, as well as efforts directed to the development of more sophisticated depot technologies.

United States Patent Number 5,925,730 (corresponding to International Patent Application PCT/US98107438, EP 1 003 774) discloses, *inter alia*, GnRH antagonist peptides according to general formula 1.

1 Ac-*D*Nal-*D*Cpa-*D*Pal-Ser-Aph(X¹)-*D*Aph(X²)-Leu-Lys(iPr)-Pro-*D*Ala-NH₂

US 5,925,730 also discloses aqueous solutions of the peptides for subcutaneous injection at concentrations of 0.75 mg/ml or 1.0 mg/ml, without danger of gelling at the point of injection.

FR-A-2 776 520 discloses solid or semi-solid pharmaceutical compositions of a soluble or gellable peptide salt which, after injection into a subject's body, form a gel providing a long-term release of the peptide.

These peptides have a high affinity for the GnRH receptor and are much more soluble in water than previously described GnRH analogues. It was suggested in the disclosure that the increased solubility of these compounds is, at least in part, responsible for the long duration of action of up to three or four days in some in vivo models. It has also been suggested that the duration of action of these compounds is dose-related, i.e. that the duration of action is dependent on the amount of peptide given. However, the optimum conditions for formulating these peptides were not discussed.

### SUMMARY OF THE INVENTION

We have now discovered that a peptide according to general formula **1** is capable of forming a gel after subcutaneous injection, and that this gel can act as a depot from which the peptide is released over a period of weeks or even months. We have also found that the key variable is the concentration of the solution rather than the amount of substance administered. The concentration of the solution must be within a functional range. If the solution is too dilute then no depot is formed and the long duration of action is lost, no matter how much drug substance is given. If the solution is too concentrated then gel formation will occur before the drug can be administered. Accordingly, in a first aspect, the present invention relates to a pharmaceutical composition for the treatment of certain disorders of the genitourinary tract and other sex-hormone dependent conditions, which composition is a solution administered by subcutaneous or intramuscular injection and provides for the continuous release of the GnRH antagonist peptide over a period (e.g. of more than two weeks). The composition may be presented as a solution that is ready for administration, but is preferably presented as a kit of parts comprising peptide (e.g. as a solid) and solvent components such that the solution can be made up immediately prior to administration. In a second aspect, the present invention provides for the use of the peptide for the prepartion of such compositions in the treatment of the disease states.

### DETAILED DESCRIPTION OF THE INVENTION

The present invention comprises a pharmaceutical composition. The composition is a solution for injection, preferably for subcutaneous injection. A first essential component of the composition is the GnRH antagonist peptide according to general formula **1**.

1 Ac-*D*Nal-*D*Cpa-*D*Pal-Ser-Aph(X¹)-*D*Aph(X²)-Leu-Lys(iPr)-Pro-*D*Ala-NH₂

In this general formula the abbreviations have the following meanings:

| | |
|---|---|
| Ac | Acetyl |
| *D*Nal | *D*-β-(2-naphthyl)alanine |
| *D*Cpa | *D*-4-chlorophenylalanine |
| *D*Pal | *D*-β-(3-pyridyl)alanine |
| Ser | Serine |
| Aph(L-Hor) | 4-aminophenylalanine wherein the ω-amino group has a substituent L-Hor |
| *D*Aph(CONH₂) | *D*-4-aminophenylalanine wherein the ω-amino group has a substituent CONH₂ |
| Leu | Leucine |
| Lys(iPr)- | N^{ω}-isopropyllysine |
| Pro | Proline |
| *D*Ala-NH₂ | *D*-alanine amide |

The substituent L-Hor is :

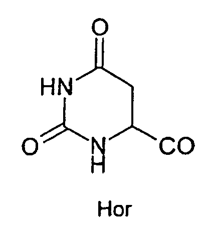

The peptide according to the above definition is capable of forming salts. In particular, it is capable of forming addition salts with acids such as hydrochloric acid, acetic acid and trifluoroacetic acid. Provided that they are pharmaceutically acceptable, all such salts are included within the scope of the present disclosure. The acetate and hydrochloride salts are particularly preferred.

A second essential component of the composition is a solvent such as water, an alcohol (for example ethanol), N-methylpyrrolidone or dimethylsulfoxide. In a preferred embodiment of the invention the solvent is water or a mixture of water and alcohol, N-methylpyrrolidone or dimethylsulfoxide such that the water constitutes at least 90% by weight of the solvent mixture. The composition may contain other components such as osmotic pressure regulating agents, for example sodium chloride and mannitol, preservatives, buffering agents and the like. In a preferred embodiment of the invention, the concentration of sodium chloride is below 2 mg/ml. In a more preferred embodiment, sodium chloride is absent from the composition and mannitol is used to adjust the osmolarity of the solution.

The composition may further include additional pharmaceutically active agents, but it is preferred that the said GnRH antagonist peptide should be the only such agent.

The composition according to the present invention may be presented in a form that is ready for immediate use, such as a solution in a sealed container or a prefilled syringe. Alternatively and preferably, the composition may be presented in a form that requires some preparation prior to administration. For example, the composition may be presented as a kit of parts, including a sealed container containing the peptide as a lyophilised powder and a second container containing the solvent or diluent. The peptide may be freeze dried. Further components may be included with the solid or liquid part. Thus the kit may comprise a first container containing the peptide and a second containing isotonic saline, or a first container containing the peptide and mannitol and a second container containing sterile water. Prior to administration the solvent is added to the container containing the peptide component in order to give the solution for injection. The kit may prevent problems caused by any lack of long term stability of solutions containing the peptide.

An essential property of the composition of the present invention is that the solution should be stable prior to administration but should convert into a gel soon (preferably immediately) after, administration. This property is a function of the concentration of the peptide. The precise concentration range effective for the purposes of the invention may vary somewhat from case to case, e.g. according to the identities of peptide and solvent and of secondary ingredient(s) when present, and to intended storage time. It is evident that in any given instance the result to be achieved and the effective concentration range therefor are directly and positively verifiable by the simplest tests and observations requiring minimal experimentation. The concentration of the peptide in solution is at least 25 mg/ml in the injectable pharmaceutical composition.

When the composition is presented as a kit of parts to be administered immediately after mixing (e.g. within 30 minutes of mixing), the peptide concentration in the final solution may be higher, for example as much as 120 mg/ml.

In a preferred embodiment of the present invention, the concentration of the peptide is not more than 80 mg/ml. In a more preferred embodiment, the concentration of the peptide is not more than 40 mg/ml.

Peptide at concentrations of for example 25mg/ml may be used to form a gel after administration which releases the peptide over a period of at least two weeks, preferably for a period of three months.

The composition according to the present invention releases the GnRH antagonist peptide into the general circulation over a period of several days, weeks, or even months. Accordingly, it causes long term blockade of the GnRH receptor, which results in a profound suppression of the release of LH and FSH. This in turn results in a suppression of gonadal function, including suppression of the release of sex steroid hormones from the gonads. Hence the compositions according to the present invention are useful in the treatment of diseases which involve stimulation of a tissue by sex steroid hormones or directly by LH or FSH. Such diseases include benign prostate hyperplasia, prostate cancer, oestrogen-dependent breast cancer, endometriosis and precocious puberty. In a second aspect, therefore, the present invention comprises a method of treating these diseases by the administration to an individual in need of such treatment of a therapeutically effective amount of a composition as described above. The compositions may also be used as contraceptive agents, particularly male contraceptive agents. When used for this purpose it may be necessary to administer testosterone in order to maintain libido. Further uses for the compositions include the regulation of ovarian function in the context of an *in vitro* fertilisation programme and as behaviour-modifying agents for the treatment of sex offenders.

In general the attending physician will decide on the details of the dosology by taking into consideration the desired therapeutic outcome and the medical history and current condition of the patient. The volume of composition administered will generally be from 1 to 10 ml, giving for example a peptide dose of 0.3 to 1200 mg. Administration will be by subcutaneous or intramuscular injection, preferably by subcutaneous injection, at a single site or divided between two or more sites. The administration will be repeated at appropriate intervals of two weeks to three months for the duration of the treatment.

The method of treatment according to the present invention may be used as the sole treatment for the disease. Alternatively, the attending physician may choose to combine the method with other treatments given simultaneously or serially. Other treatments may include the administration of other pharmaceutical agents, including those acting by mechanisms independent of the GnRH-LH/FSH-gonad pathway, and non-pharmaceutical treatments such as surgery.

In a further aspect, the present invention provides a use for the GnRH antagonist peptides, which use is as a component for the manufacture of a pharmaceutical composition as described above.

The present invention is illustrated further in the following non-limiting Examples.

### EXAMPLES

### Example 1 - Preparation of peptides

The peptides used in the compositions of the present invention can be prepared according to the methods described in US Patent No. 5,925,730. In particular, the peptide Ac-*D*Nal-*D*Cpa-*D*Pal-Ser-Aph(L-Hor)-*D*Aph(CONH₂)-Leu-Lys(iPr)-Pro-*D*Ala-NH₂ ("Peptide 1") was prepared according to the method of Example 1 of the US patent and isolated as its acetate salt.

### Example 2 - Stability of aqueous solution

Peptide 1 was dissolved in water at various concentrations, and the resulting solutions were allowed to stand at room temperature for an extended period of time. Gel formation was determined by visual examination. The observations are summarised in Table 1.

| **Table 1 - Stability of aqueous solutions** | |
|---|---|
| ***Concentration* (mg*/*ml)*** | ***Stability*** |
| 0.25 | No gel formation after 6 months |
| 1.0 | No gel formation after 6 months |
| 5.0 | Gel formation after 4 weeks |
| 10.0 | Gel formation after 2 weeks |
| 30.0 | Gel formation after 48 hours |
| 40.0 | Gel formation after 24 hours |
| 60.0 | Gel formation after 8 hours |
| 80.0 | Rapid gel formation within 60 minutes |
| 120.0 | Rapid gel formation within 30 minutes |

| | |
|---|---|
| * calculated as free base | |

### Example 3 - Minimum concentration needed to form gel in vivo

Peptide 1 was dissolved in 5% mannitol at various concentrations and injected subcutaneously into rats. The animals were sacrificed after 24 hours and the injection site was dissected and examined. When deposits of gel were found these were removed and weighed to assess completeness of gel formation. Significant gel formation was observed with concentrations of peptide greater than 0.3 mg/ml.

### Example 4 - Efficacy of formulation in vivo

Peptide 1 is dissolved in 5% mannitol (25 mg/ml). Three ovariectomised Rhesus monkeys are treated with this solution (80 µl/kg) by subcutaneous injection. Serum LH levels is measured for the following 101 days. The results are summarised in Table 2.

| **Table 2 - Biological action** | |
|---|---|
| ***Time*** | ***Serum LH (ng*/*ml), mean ± se*** |
| 0 | 60.1 ± 7.5 |
| Hour 6 | 16.2 ± 1.9 |
| Day 1 | 10.5 ± 1.5 |
| Day 2 | 11.8 ± 2.6 |
| Day 7 | 6.7 ± 1.2 |
| Day 14 | 5.8 ± 0.9 |
| Day 21 | 6.6 ± 1.0 |
| Day 28 | 9.4 ± 1.3 |
| Day 35 | 8.8 ± 1.0 |
| Day 42 | 11.8 ± 2.3 |
| Day 72 | 29.5 ± 4.3 |
| Day 101 | 48.9 ± 8.3 |

### Example 5 - Compositions according to the invention

### 5A - Solution for injection

A solution is prepared by dissolving 51.84 g of the peptide Ac-DNal-DCpa-DPal-SerAph(L-Hor)-DAph(CONH₂)-Leu-Lys(iPr)-Pro-DAla-NH₂ acetate (Peptide 1, see Example 1) and 500 g of mannitol in 10 litres of sterile water to give a final concentration of 5 mg/ml of peptide (calculated as the free base) in 5% aqueous mannitol. The solution is filtered through a 0.2-micron filter and divided into 5000 glass vials to provide 5000 individual doses of the solution, each of 2 ml.

### 5B - Two-component kit

A solution is prepared by dissolving 414.7 g of the peptide Ac-DNal-DCpa-DPal-SerAph(L-Hor)-DAph(CONH₂)-Leu-Lys(iPr)-Pro-DAla-NH₂ acetate (Peptide 1, see Example 1) and 250 g of mannitol in 10 litres of sterile water. The solution is filtered through a 0.2-micron filter and divided into 5000 glass vials, then frozen and lyophilised.

A second solution is prepared by dissolving 250 g of mannitol in 10 litres of sterile water. This solution is filtered through a 0.2-micron filter and divided into 5000 glass vials. A kit is then made up of one vial of lyophilisate and one of mannitol solution, such that when the lyophilisate is dissolved in the mannitol solution prior to administration there results a 2 ml dose of a 40 mg/ml solution of the peptide in 5% aqueous mannitol.

The data presented in Example 2 establishes a maximum concentration above which the peptides form gels too rapidly to be conveniently administered in a clinical situation. Example 3 establishes a minimum concentration below which the peptides do not form gels following administration and so would not give the desired long duration of action. Example 4 demonstrates that the compositions according to the present invention are effective in blocking the release of LH and testosterone in an animal model. Such results are widely acceptable as an indicator of clinical efficacy in human steroid dependent pathologies. Hence they are illustrative of the clinical utility of the compositions of the invention such as, but not limited to, those of Example 5.

Analysis of the pharmacokinetics of Peptide 1 after subcutaneous or intramuscular administration to beagle dogs indicates that if high initial concentrations are necessary in clinical setting, it may be achieved by administering the dose intramuscularly as opposed to subcutaneously. If the focus is to obtain an extended release profile, a subcutaneous injection will lead to a higher fraction of the total dose being absorbed (almost twice as high as compared to intramuscular administration).

## Claims

1. An injectable pharmaceutical composition comprising a solution in a pharmaceutically acceptable solvent of a GnRH antagonist peptide according to general formula (1) or a pharmaceutically acceptable salt thereof: the concentration of the peptide in the solution being at least 25 mg/ml such that the peptide is not in gel form but forms a gel after injection.

2. The composition according to claim 1 wherein the concentration of the peptide in the solution is not more than 120 mg/ml.

3. The composition according to claim 1 or 2 wherein the concentration of the peptide in the solution is not more than 80 mg/ml.

4. The composition according to any preceding claim wherein the concentration of the peptide is not more than 40 mg/ml.

5. The composition according to any preceding claim wherein the solvent is water or a mixture of water and a second solvent such that at least 90 % by weight of the solvent is water.

6. The composition according to any preceding claim wherein the peptide is in the form of its hydrochloride or acetate salt.

7. The composition according to any preceding claim which is for the treatment of benign prostate hyperplasia, prostate cancer, oestrogen-dependent breast cancer, endometriosis or precocious puberty, for use as a contraceptive agent or in an *in vitro* fertilisation programme, or for the treatment of sex offenders.

8. Use of a GnRH antagonist peptide according to general formula (1) or a pharmaceutically acceptable salt thereof: for the preparation of a pharmaceutical composition for the treatment of benign prostate hyperplasia, prostate cancer, oestrogen-dependent breast cancer, endometriosis or precocious puberty, for providing contraception, for controlling ovarian function in an *in vitro* fertilisation programme, or for the treatment of sex offenders, where the composition comprises a solution of the GnRH antagonist peptide in a pharmaceutically acceptable solvent at a concentration of the peptide of at least 25 mg/ml and the composition is to be injected subcutaneously or intramuscularly such that the peptide spontaneously forms a gel after administration and the gel acts as a depot which releases the peptide over a period of at least two weeks.

## Patentansprüche

1. Injizierbare pharmazeutische Zusammensetzung, umfassend eine Lösung eines GnRH-antagonistischen Peptids gemäß der allgemeinen Formel (1) oder eines pharmazeutisch verträglichen Salzes davon in einem pharmazeutisch verträglichen Lösungsmittel: wobei die Konzentration des Peptids in der Lösung mindestens 25 mg/ml ist, so dass das Peptid nicht in Gelform vorliegt, aber nach Injektion ein Gel bildet.

2. Zusammensetzung gemäß Anspruch 1, wobei die Konzentration des Peptids in der Lösung nicht mehr als 120 mg/ml ist.

3. Zusammensetzung gemäß Anspruch 1 oder 2, wobei die Konzentration des Peptids in der Lösung nicht mehr als 80 mg/ml ist.

4. Zusammensetzung gemäß einem der vorhergehenden Ansprüche, wobei die Konzentration des Peptids nicht mehr als 40 mg/ml ist.

5. Zusammensetzung gemäß einem der vorhergehenden Ansprüche, wobei das Lösungsmittel Wasser oder ein Gemisch aus Wasser und einem zweiten Lösungsmittel ist, so dass mindestens 90 Gew.% des Lösungsmittels Wasser ist.

6. Zusammensetzung gemäß einem der vorhergehenden Ansprüche, wobei das Peptid in der Form seines Hydrochlorid- oder Acetatsalzes vorliegt.

7. Zusammensetzung gemäß einem der vorhergehenden Ansprüche für die Behandlung von gutartiger Prostata-Hyperplasie, Prostatakrebs, Östrogen-abhängigem Brustkrebs, Endometriose oder frühzeitiger Pubertät, zur Verwendung als Verhütungsmittel oder bei einem in vitro Fertilisierungsprogramm oder zur Behandlung von Sexualstraftätern.

8. Verwendung eines GnRH-antagonistischen Peptids gemäß der allgemeinen Formel (1) oder eines pharmazeutisch verträglichen Salzes davon: zur Herstellung einer pharmazeutischen Zusammensetzung zur Behandlung von gutartiger Prostata-Hyperplasie, Prostatakrebs, Östrogen-abhängigem Brustkrebs, Endometriose oder frühzeitiger Pubertät, zur Empfängnisverhütung, zur Kontrolle der Eierstockfunktion in einem in vitro Fertilisierungsprogramm oder zur Behandlung von Sexualstraftätern, wobei die Zusammensetzung eine Lösung des GnRH-antagonistischen Peptids in einem pharmazeutisch verträglichen Lösungsmittel bei einer Konzentration des Peptids von mindestens 25 mg/ml umfasst und die Zusammensetzung subkutan oder intramuskulär zu injizieren ist, so dass das Peptid spontan nach der Verabreichung ein Gel bildet und das Gel als Depot wirkt, das das Peptid über einen Zeitraum von mindestens zwei Wochen abgibt.

## Revendications

1. Composition pharmaceutique injectable comprenant une solution dans un solvant pharmaceutiquement acceptable d'un peptide antagoniste de la GnRH selon la formule générale (1) ou un de ses sels pharmaceutiquement acceptables: la concentration du peptide dans la solution étant d'au moins 25 mg/ml de façon que le peptide ne soit pas sous forme de gel, mais forme un gel après injection.

2. Composition selon la revendication 1, dans laquelle la concentration du peptide dans la solution est d'au plus 120 mg/ml.

3. Composition selon la revendication 1 ou 2, dans laquelle la concentration du peptide dans la solution est d'au plus 80 mg/ml.

4. Composition selon l'une quelconque des revendications précédentes, dans laquelle la concentration du peptide est d'au plus 40 mg/ml.

5. Composition selon l'une quelconque des revendications précédentes, dans laquelle le solvant est l'eau ou un mélange d'eau et d'un deuxième solvant de façon qu'au moins 90 % en masse du solvant soit de l'eau.

6. Composition selon l'une quelconque des revendications précédentes, dans laquelle le peptide est sous forme de son chlorhydrate ou de son acétate.

7. Composition selon l'une quelconque des revendications précédentes, qui est destinée au traitement de l'hyperplasie bénigne de la prostate, du cancer de la prostate, du cancer du sein dépendant des oestrogènes, de l'endométriose ou de la puberté précoce, à une utilisation comme agent contraceptif ou dans un programme de fécondation *in vitro*, ou au traitement de délinquants sexuels.

8. Utilisation d'un peptide antagoniste de la GnRH de formule générale (1) ou d'un de ses sels pharmaceutiquement acceptables: pour la préparation d'une composition pharmaceutique destinée au traitement de l'hyperplasie bénigne de la prostate, du cancer de la prostate, du cancer du sein dépendant des oestrogènes, de l'endométriose ou de la puberté précoce, à la contraception, au contrôle de la fonction ovarienne dans un programme de fécondation *in vitro,* ou au traitement de délinquants sexuels, dans laquelle la composition comprend une solution du peptide antagoniste de la GnRH dans un solvant pharmaceutiquement acceptable à une concentration du peptide d'au moins 25 mg/ml et la composition doit être injectée par voie sous-cutanée ou intramusculaire de façon que le peptide forme spontanément un gel après l'administration et que le gel ait un effet retard qui libère le peptide sur une période d'au moins 2 semaines.
